# FASCICULE DE BREVET EUROPEEN

(11) **EP 3 256 056 B1**
(45) Date de publication et mention de la délivrance du brevet: **04.12.2024**
(21) Numéro de dépôt: 16707917.7
(22) Date de dépôt: 12.02.2016
(51) Int. Cl.: A61B 17/064, A61B 17/068

(54) **SYSTÈME DE CHIRURGIE ENDOSCOPIQUE FORMÉ D'UNE PART PAR UNE PLURALITÉ D'AGRAFES ET D'AUTRE PART PAR UN APPLICATEUR ENDOSCOPIQUE**
ENDOSKOPISCHES CHIRURGISCHES SYSTEM AUS EINER MEHRZAHL VON KLAMMERN UND ENDOSKOPISCHEM APPLIKATOR
ENDOSCOPIC SURGERY SYSTEM CONSISTING OF A PLURALITY OF STAPLES AND AN ENDOSCOPIC APPLICATOR

(30) Priorité: 13.02.2015 FR 1551204
(43) Date de publication de la demande: 20.12.2017
(73) Titulaire: TAURUS ENDOSCOPY, 67000 Strasbourg (FR)
(72) Inventeur: ALZAGA, Amilcar, 03710 Mexico D.F. (MX); RIVA, Pietro, 20811 Cesano Maderno (MB) (IT)
(74) Mandataire: IP Trust
(86) Numéro de dépôt international: PCT/FR2016/050330
(87) Numéro de publication internationale: WO 2016/128693

(56) Documents cités:
- WO-A1-02/19920
- US-A- 5 395 030
- US-A1- 2009 072 006
- US-A1- 2009 272 783
- US-A1- 2009 272 786
- US-A1- 2009 275 957

## Description

### Domaine de l'invention

La présente invention concerne le domaine des applicateurs d'agrafe endoscopique. Un tel applicateur permet aux chirurgiens de poser des agrafes afin de maintenir des tissus pendant une intervention par voie endoscopique.

Un applicateur d'agrafes endoscopique est conçu pour retenir une pluralité d'agrafes, notamment des agrafes de ligature dans un canal à agrafes et un système de mâchoires servant à appliquer une agrafe. Le système de mâchoires est relié à l'extrémité distale du canal à agrafes et à l'extrémité proximale à une poignée de commande.

### Etat de la technique

On connaît dans l'état de la technique le brevet US8133240 décrivant un système d'agrafage endoscopique. Ce système comprend des agrafes déformables disposées bout à bout dans une cavité d'un canal intérieur. Des glissières coulisse à l'intérieur de ce canal sous l'action d'élément de poussée.

Lors de son éjection, l'agrafe déformée pour serrer le tissu corporel.

On connaît aussi le brevet EP0713684 décrivant un système formé par une cartouche d'agrafes et un instrument d'agrafage pour appliquer une ou plusieurs agrafes chirurgicales à un tissu. Chaque agrafe a un corps en forme générale de U comportant une traverse supérieure ayant des pattes d'agrafes attenantes sur les côtés opposés de la traverse supérieure.

Un boîtier de cartouche recevant une pluralité d'agrafes est agencé dans une rangée pour qu'elles aient un déplacement longitudinal à travers celui-ci. Une enclume est montée sur ledit boîtier.

L'agrafe la plus en avant est déplacée jusqu'au contact avec ladite enclume puis basculée depuis une orientation transversale jusqu'à une orientation longitudinale, puis déformée par une pression sur ladite enclume pour fixer l'agrafe.

On connaît aussi la demande de brevet US2009272786 décrivant une agrafeuse chirurgicale qui comprend une tige tubulaire allongée s'étendant de manière distale à partir de la poignée. La tige tubulaire comprend une extrémité proximale fixée sur la poignée et une extrémité distale dans laquelle est formée une ouverture de déploiement. Un ensemble de déploiement d'agrafe est disposé à l'intérieur de la tige pour évacuer des agrafes depuis l'ouverture de déploiement située à l'extrémité distale de l'arbre, l'ensemble de déploiement d'agrafe supportant une agrafe de sorte qu'un axe longitudinal de l'agrafe soit aligné avec un axe longitudinal de la tige.

On connaît aussi le brevet US5395030 qui décrit une agrafeuse chirurgicale qui comprend une section d'application d'agrafes contenant une pluralité d'agrafes, un poussoir d'agrafes incorporé dans la section d'application d'agrafes, pour appliquer les agrafes, une section d'enclume pour déformer les agrafes, afin d'agrafer les tissus corporels ensemble, une section d'opération pour actionner le poussoir d'agrafes et la section d'enclume, une fenêtre d'observation située près de la section d'enclume, et une section oculaire pour afficher des images fournies par la fenêtre d'observation.

On connaît aussi la demande de brevet US2009272783 décrivant un dispositif d'agrafage chirurgical pour déployer des agrafes reliées par une suture à un tissu comprend une poignée ayant un mécanisme d'actionnement d'agrafe, une tige de support tubulaire ayant un axe longitudinal et s'étendant à partir de la poignée, et une cartouche d'agrafes montée sur la tige de support et reliée à l'actionneur d'agrafe mécanisme pour appliquer une ou plusieurs agrafes sur le tissu. La cartouche a un logement de cartouche adapté pour recevoir une pluralité d'agrafes dans une rangée pour un mouvement longitudinal à travers celui-ci et une enclume montée sur le logement. La cartouche comprend en outre un mécanisme pour faire avancer l'agrafe la plus en avant de la rangée en engagement avec l'enclume, et un guide de suture supportant une longueur de suture qui s'étend dans une boucle pour la fixation aux agrafes lorsqu'elles sont fixées au tissu.

On connaît aussi la demande de brevet US2009072006 concernant une agrafeuse chirurgicale peut comprendre au moins une agrafe, un poussoir et un bras évasé ; où le mouvement d'au moins l'un parmi le bras évasé et le poussoir par rapport à l'autre amène le bras évasé à entrer en contact puis à évaser au moins une agrafe.

On connaît aussi la demande de brevet US2009275957 qui concerne un système d'apposition de tissu comprenant un ensemble de mise en place auquel est fixée sélectivement une pince à tissu pour le déploiement et l'application de la pince à tissu. La pince à tissu est formée et dimensionnée pour s'engager dans des emplacements espacés le long d'une étendue de tissu et, lors de sa rotation, rapprocher les emplacements espacés en apposition pour créer ainsi un pli de tissu.

### Inconvénients de l'art antérieur

Les solutions de l'art antérieur ne sont pas totalement satisfaisantes car elles prévoient généralement un positionnement de l'agrafe appliquée dans le plan transversal, ce qui réduit l'ouverture des pointes de l'agrafe venant accrocher les bords des tissus à maintenir.

Par ailleurs, le chargeur d'agrafes présente souvent un encombrement longitudinal assez important, rendant la solution mal adaptée à l'utilisation avec des endoscopes souples.

Enfin, les solutions de l'art antérieur masquent généralement une grande partie du champ de vision endoscopique, ce qui rend ces interventions délicates particulièrement malaisées.

### Solution apportée par l'invention

Afin de remédier à ces inconvénients, la présente invention concerne selon son acception la plus générale un système de chirurgie endoscopique présentant les caractéristiques énoncées dans la revendication 1.

### Description détaillée d'un exemple non limitatif de réalisation

La présente invention sera mieux comprise à la lecture de la description qui suit, se référant aux dessins annexés où :
- la figure 1 représente une vue de l'organe de commande chargé avec une agrafe
- la figure 2 représente une vue du chargeur d'agrafes
- les figures 3 à 6 représentent des vues de l'applicateur à différentes étapes de fonctionnement.

### Description de l'organe de commande

L'organe de commande comprend un manchon tubulaire (1) creux rigide. Une tige rigide (2) de section transversale polygonale coulisse longitudinalement à l'intérieur dans ce manchon (1) présentant un canal de guidage à section carrée complémentaire.

Ce manchon (1) présente à son extrémité distale un chanfrein (3) définissant une surface inclinée par rapport à un plan longitudinal, aboutissant à un téton (4) s'étendant perpendiculairement à l'axe longitudinal. Le chanfrein (3) et le téton (4) forment une zone de réception d'une agrafe (5) présentant une forme générale de « fer à cheval ».

### Description de l'agrafe

L'agrafe présente une forme, en vue de face et avant agrafage, une forme générale semi-annulaire ou de « fer à cheval». Dans l'exemple décrit, l'agrafe est formée par une découpe en acier mais elle peut aussi être formée par mise en forme d'un fil métallique ou en un matériau biodégradable.

Elle présente deux branches arquées (8, 9) s'étendant dans un plan transversal perpendiculaire à l'axe longitudinal du manchon (1), symétriquement par rapport à un plan médian (6) passant par le milieu d'un tronçon de liaison (7). Ce plan médian (6) est défini par un axe parallèle à l'axe longitudinal du manchon (1) et par l'axe du téton (4).

Le tronçon de liaison (7) désigne simplement la partie comprise entre les deux branches (8, 9). Il n'y a pas de séparation entre cette zone qualifiée de tronçon de liaison (7) et les branches (8, 9) dans l'exemple décrit.

Mais il est envisageable dans d'autres formes de réalisation de prévoir une zone (7) reliée des lignes de pliage aux branches (8, 9).

Ce tronçon médian (7) présente une forme semi-tubulaire et est réalisée par déformation du matériau vers l'arrière avec une matrice cylindrique. La forme semi-tubulaire du tronçon médian (7) permet le positionnement d'un organe de guidage prévu à l'extrémité d'un instrument endoscopique, afin de faciliter la manipulation de l'agrafe au moment de sa pose.

Chacune des deux branches (8, 9) présente une extrémité pointue (10, 11) en forme de crochet, s'étendant en avant du plan transversal contenant la branche correspondante (8, 9).

La tangente à l'extrémité pointue (11, 21) forme, par rapport à la normale au plan transversal, un angle compris supérieur à 0° et inférieur à 90°, et de préférence compris entre 5° et 50°. Cette extrémité pointue permet d'accrocher les tissus au voisinage de la zone de pose de l'agrafe, et à exercer une traction latérale pour les rapprocher avant l'agrafage. Lorsque l'angle formé par la tangente est important, on favoriser l'entraînement des tissus sans les perforer. Si l'angle est plus faible, on favorise la pénétration de l'extrémité pointue dans les tissus.

Les deux branches (8, 9) portent également, chacune, un crochet (12, 13) se projetant en avant du plan transversal, et positionné plus près de l'extrémité pointue (10, 11) que dudit tronçon de liaison (7).

L'extrémité pointue d'accrochage (10, 11) et le crochet (12, 13) peuvent être réalisés pour former une extrémité fendue de la branche (8, 9), une des langues de cette extrémité fendue formant la pointe d'accrochage (10, 11) et l'autre formant le crochet (12, 13).

Dans l'exemple décrit, la longueur déroulée de la pointe d'accrochage (10, 11) et sensiblement égale à la longueur déroulée du crochet (12, 13).

Ce crochet (12, 13) présente une surface de pincement (14, 15) sensiblement parallèle au plan transversal. Cette surface de pincement (14, 15) viendra s'appuyer de part et d'autre des tissus au moment de l'agrafage, pour assurer leur maintien sans les perforer.

Le fonctionnement de l'agrafe est le suivant : l'agrafe est positionnée contre les deux lèvres à agrafer avec un applicateur. L'agrafe est en position ouverte, et les deux extrémités pointues (10, 11) viennent affleurer les tissus de part et d'autre de la ligne de séparation des deux lèvres. Ces extrémités pointues (10, 11) pénètrent légèrement dans les tissus et les accrochent pour assurer leur rapprochement lorsque l'on commence à refermer l'agrafe par un instrument faisant pivoter les deux branches (8, 9) par rapport à l'axe médian passant par la partie tubulaire de la zone transversale (7). Les extrémités pointues (10, 11) effectuent alors un mouvement de balayage en arc de cercle, ce qui ramène les tissus au bord des lèvres entre les deux branches, entre les deux crochets (12, 13). Lorsque les deux branches sont rabattues l'une contre l'autre, les surfaces de pincement (14, 15) maintiennent les bords des lèvres en position.

### Interaction entre le manchon (2) et l'agrafe (5)

Le manchon (2) présente à son extrémité frontale distale deux encoches latérales (3, 4) dont la hauteur correspond à la largeur des branches (8, 9) de l'agrafe (5).

Par ailleurs, la hauteur de la zone de liaison (7) est légèrement inférieure à la section du canal formé dans le tube (1).

Le téton (4) présente un rayon de courbure correspondant sensiblement au rayon de courbure intérieur de la zone de liaison (7).

Lorsque la tige (2) est déplacée longitudinalement par rapport au manchon (1), l'agrafe (5) est appliquée contre la paroi frontale distale du manchon (1) et les deux branches (8, 9) viennent s'engager dans les encoches latérales (3, 4). En continuant l'application d'un effort longitudinal, on déforme ces branches (8, 9), ce qui provoque une fermeture de l'agrafe. Les pointes (11, 12) accrochent et déplacent les tissus, qui viennent se positionner entre les surfaces de pincement (14, 15) des crochets (11, 12) pour assurer le maintien des tissus.

### Description du chargeur d'agrafes

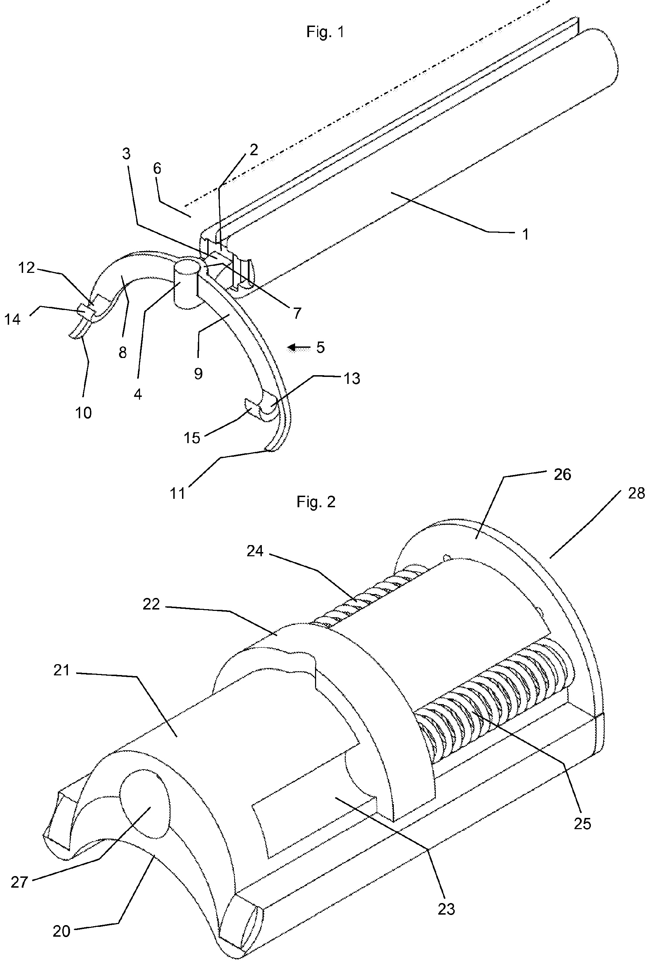

La figure 2 représente un exemple de réalisation d'un chargeur multi-agrafe pour un applicateur.

Il est constitué par un boîtier (20) en forme de tuile, présentant une surface ventrale complémentaire à la surface extérieure d'un endoscope. Il peut ainsi être adapté sur un endoscope standard, afin de bénéficier des moyens d'éclairage et d'examen visuel, et éventuellement d'aspiration, intégrés dans un tel endoscope.

La partie dorsale (21) présente une forme semi-tubulaire, définissant une surface enveloppante dont la section correspond à la section transversale définie par les bords intérieurs des deux branches (8, 9) d'une agrafe (5). Les agrafes peuvent ainsi être positionnés sur cette partie dorsale (21). Un arceau (22) est engagé sur cette partie dorsale (21) et guidé par deux cannelures latérales (23). Cet arceau (22) est mobile longitudinalement, sous l'action de deux ressorts (24, 25), s'appuyant sur une collerette frontale proximale (26).

Le chargeur présente par ailleurs un logement tubulaire (27) s'étendant longitudinalement, pour l'introduction du manchon (1) précédemment décrit.

Les agrafes sont toutes placées transversalement et de manière adjacente pour former une série d'agrafes (5) parallèles entre-elles.

La zone de liaison (7) présente une surépaisseur voisine à celle formée par les crochets (13, 14), ce qui permet de maintenir un parallélisme entre les agrafes consécutives.

### Fonctionnement de l'applicateur

Le chargeur (28) est placé à cheval sur l'extrémité distale d'un endoscope (29). La tige (2) est repoussée légèrement hors du manchon (1).

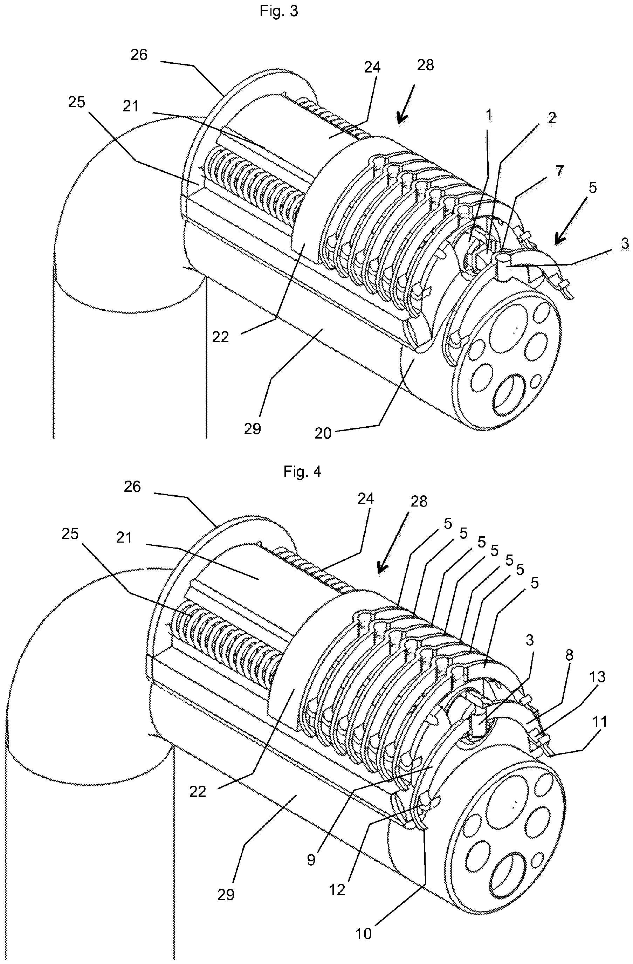

L'agrafe (5) à l'extrémité frontale distale de la série d'agrafes adjacentes est repoussée par l'action de l'arceau (22) et vient se loger dans la zone de réception de la tige (2) comme représenté en figure 3. Les autres agrafes sont maintenues en place autour de la zone dorsale (21) du chargeur (28) par frottement. La zone de liaison (7) est placée derrière le téton (4), c'est-à-dire du côté de l'extrémité proximale (du côté de l'opérateur), alors que le téton est du côté de l'extrémité distale (du côté des tissus à maintenir).

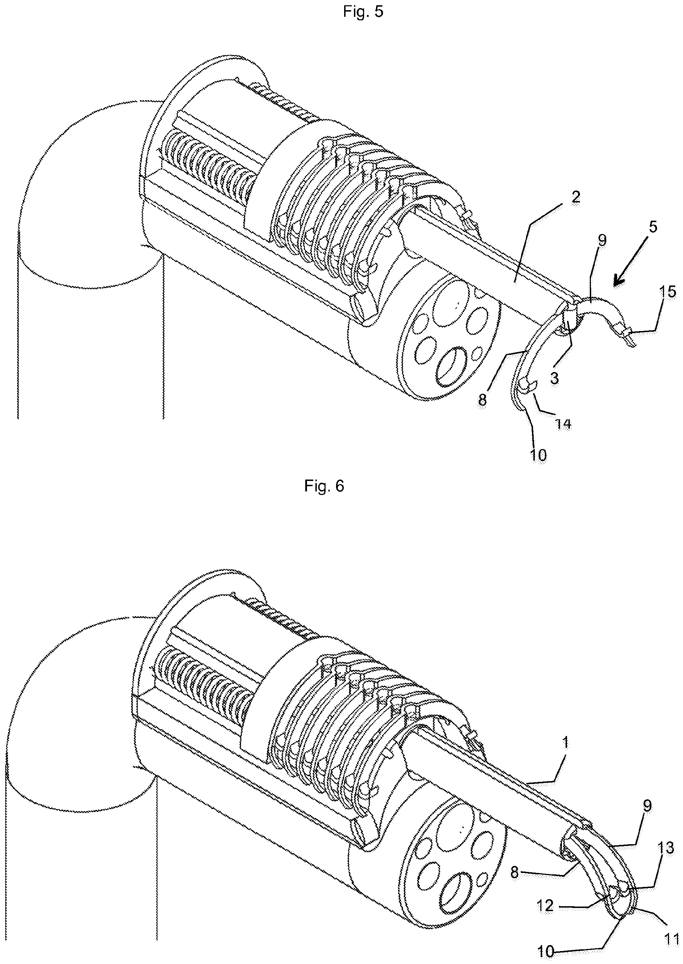

On retire ensuite la tige (2) à l'intérieur du manchon (1) comme représenté en figure 4. Les branches (8, 9) s'engagent alors dans les encoches latérales prévues à l'extrémité frontale du manchon (1). L'agrafe (5) est alors coincée entre le téton (4) et la surface frontale du manchon (1), et peut être manipulé par le déplacement conjoint du manchon (1) et de la tige (2). On peut ainsi déplacer l'agrafe par une action extérieure en déplacement axial longitudinal et en rotation autour de l'axe longitudinal comme représenté en figure 5. Pendant le déplacement longitudinal, les pointes (10, 11) viennent affleurer les tissus, dans le champ de vision de l'endoscope, et accrochent les tissus à rapprocher.

En déplaçant ensuite le tube (2) par rapport au manchon (1), ce qui produit la déformation des deux branches (8, 9) qui viennent se replier pour venir en position parallèle, pour pincer les tissus entre les crochets (12, 13). Pendant que les branches (8, 9) sont rabattues l'une contre l'autre, les extrémités pointues (10, 11) entraînent les tissus selon une trajectoire semi-circulaire, pour les positionner entre les crochets (12, 13).

L'utilisateur peut ensuite charger une autre agrafe sur la tige mobile en rétractant le manchon et la tige et recommencer ainsi le cycle pour la pose d'une nouvelle agrafe.

Grâce au moyen de déplacement longitudinal de l'agrafe et à la position transversale de l'agrafe (5) une fois celle-ci chargée sur le moyen de retenue, l'applicateur peut avantageusement être disposé sur un endoscope en retrait de l'extrémité de ce dernier afin que le dispositif ne pénètre pas dans le champ de vision de l'endoscope mais que les extrémités des branches (8,9) de l'agrafe (5) pénètrent dans le champ de vision dudit endoscope.

## Revendications

1. - Système de chirurgie endoscopique formé par des agrafes présentant une zone centrale et deux branches (8, 9) et par un applicateur endoscopique d'agrafes comportant un chargeur d'agrafes (28) disposées transversalement,
- l'applicateur comportant un manchon tubulaire (1) creux rigide et une tige rigide (2) coulissant longitudinalement à l'intérieur dans ce manchon (1) présentant un canal de guidage à section complémentaire,
- lesdites agrafes présentant, en vue de face et avant agrafage, une forme générale semi-annulaire ou de « fer à cheval », et étant formées par deux branches arquées (8, 9) reliées par un tronçon de liaison (7) et s'étendant dans un plan transversal perpendiculaire à l'axe longitudinal du manchon (1), symétriquement par rapport à un plan médian (6) passant par le milieu dudit tronçon de liaison (7), chacune des branches arquées (8, 9) présentant une extrémité pointue (10, 11) en forme de crochet s'étendant en avant du plan transversal contenant la branche correspondante (8, 9), et un crochet (12, 13) se projetant en avant dudit plan transversal et positionné plus près de ladite extrémité pointue respectivement (10, 11) que ledit tronçon de liaison (7), lesdits crochets (12, 13) présentant une surface de pincement (14, 15) sensiblement parallèle audit plan transversal,
- ladite tige (2) présentant un moyen de retenue (3) d'une agrafe à appliquer (5) disposée dans un plan transversal par rapport audit moyen de retenu (3) pour assurer sa déformation par pliage par rapport au plan médian (6) de symétrie de ladite agrafe.

2. - Système de chirurgie endoscopique selon la revendication précédente **caractérisé en ce que** ladite tige (2) présente une section transversale polygonale complémentaire de la section dudit canal de guidage prévue dans le manchon (1).

3. - Système de chirurgie endoscopique selon la revendication précédente **caractérisé en ce que** ledit chargeur d'agrafes (28) comporte un boîtier (20) apte à être monté sur l'extrémité distale d'un endoscope (29), et présente une partie dorsale définissant une surface de réception dont la section correspond à la section transversale définie par les bords intérieurs des deux branches d'une agrafe, le chargeur comportant en outre un arceau engagé sur cette partie dorsale et guidé par deux cannelures latérales.

## Patentansprüche

1. Endoskopisches Chirurgiesystem, das aus Klammern, die eine mittlere Zone und zwei Schenkel (8, 9) aufweisen, und aus einem endoskopischen Klammerapplikator ausgebildet ist, der eine Ladeeinrichtung (28) für quer angeordnete Klammern umfasst, wobei der Applikator eine starre hohle rohrförmige Hülse (1) und einen starren Stab (2) umfasst, der in Längsrichtung im Inneren dieser Hülse (1) gleitet, die einen Führungskanal mit komplementärem Abschnitt aufweist,
- wobei die Klammern in der Vorderansicht und vor dem Zusammenklammern eine allgemeine Halbring- oder "Hufeisen"-Form besitzen und aus zwei bogenförmigen Schenkeln (8, 9) ausgebildet sind, die durch ein Verbindungsstück (7) verbunden sind und sich in einer Querebene senkrecht zu der Längsachse der Hülse (1) erstrecken, symmetrisch in Bezug auf eine Mittelebene (6), die durch das Zentrum des Verbindungsstückes (7) verläuft, wobei jeder der bogenförmigen Schenkel (8, 9) ein spitzes Ende (10, 11) in Form eines Hakens, der sich vor der Querebene erstreckt, die den entsprechenden Schenkel (8, 9) enthält, und einen Haken (12, 13) aufweist, der von der Querebene vorsteht und jeweils näher an diesem spitzen Ende (10, 11) als das Verbindungsstück (7) positioniert ist, wobei die Haken (12, 13) eine Greifoberfläche (14, 15) aufweisen, die im Wesentlichen parallel zu der Querebene ist,
- wobei der Stab (2) ein Haltemittel (3) einer zu applizierenden Klammer (5), die in einer Querebene relativ zu dem Haltemittel (3) angeordnet ist, zum Gewährleisten dessen Verformung durch Falten relativ zu der symmetrischen Mittelebene (6) der Klammer aufweist.

2. Endoskopisches Chirurgiesystem nach dem vorstehenden Anspruch, **dadurch □gekennzeichnet, □dass** der Stab (2) einen polygonalen Querschnitt aufweist, der komplementär zu dem Abschnitt des Führungskanals ist, der in der Hülse (1) vorgesehen ist.

3. Endoskopisches Chirurgiesystem nach dem vorstehenden Anspruch, **dadurch □gekennzeichnet, □dass** die Ladeeinrichtung (28) für Klammern ein Gehäuse (20) umfasst, das geeignet ist, um an dem distalen Ende eines Endoskops (29) montiert zu werden, und einen dorsalen Teil aufweist, der eine Aufnahmeoberfläche definiert, deren Abschnitt dem Querschnitt entspricht, der durch die Innenkanten der zwei Schenkel einer Klammer definiert wird, wobei die Ladeeinrichtung ferner einen Bogen umfasst, der an diesem dorsalen Teil befestigt ist und durch zwei seitliche Rillen geführt wird.

## Claims

1. Endoscopic surgery system formed by staples having a central zone and two limbs (8, 9) and by an endoscopic staple applicator comprising a feeder (28) for transversely arranged staples, the applicator comprising a rigid hollow tubular sleeve (1) and a rigid rod (2) sliding longitudinally inside this sleeve (1) which has a guide channel with a complementary cross-section,
- said staples having, in front view and before stapling, a general semi-annular or "horseshoe" shape, and being formed by two arcuate limbs (8, 9) connected by a connecting portion (7) and extending in a transverse plane perpendicular to the longitudinal axis of the sleeve (1), symmetrically with respect to a median plane (6) passing through the middle of said connecting portion (7), each of the arcuate limbs (8, 9) having a hook-shaped pointed end (10, 11) extending forward from the transverse plane containing the corresponding limb (8, 9), and a hook (12, 13) projecting forward from said transverse plane and positioned closer to said pointed end (10, 11) respectively than said connecting portion (7), said hooks (12, 13) having a gripping surface (14, 15) substantially parallel to said transverse plane,
- said rod (2) having a means (3) for retaining a staple (5) to be applied which is arranged in a plane transverse to said retaining means (3), in order to ensure the deformation thereof by bending relative to the median plane (6) of symmetry of said staple.

2. Endoscopic surgery system according to the preceding claim, **characterized in that** said rod (2) has a polygonal cross-section complementary to the cross-section of said guide channel provided in the sleeve (1).

3. Endoscopic surgery system according to the preceding claim, **characterized in that** said staple feeder (28) comprises a housing (20) suitable for being mounted on the distal end of an endoscope (29), and has a dorsal part defining a receiving surface, the cross-section of which corresponds to the cross-section defined by the inner edges of the two limbs of a staple, the feeder further comprising an arch engaged on this dorsal part and guided by two lateral flutes.
